# EUROPEAN PATENT APPLICATION

(11) **EP 2 716 233 A1**
(43) Date of publication of application: **09.04.2014**
(21) Application number: 12792055.1
(22) Date of filing: 31.05.2012
(51) Int. Cl.: A61B 8/08

(54) **ULTRASONIC PROBE, BIOINFORMATION MEASUREMENT DEVICE, AND BIOINFORMATION MEASUREMENT METHOD**

(30) Priority: 01.06.2011 JP 2011123383
(71) Applicant: Kuroki, Shigehiro, Tochigi 329-0433 (JP); Sanyo-Souken Ltd., Chiba 272-0835 (JP)
(72) Inventor: YAMASHITA, Shiro, Ichikawa-shi Chiba 272-0835 (JP); KUROKI, Shigehiro, Shimotsuke-shi Tochigi 3290433 (JP)
(74) Representative: Müller-Boré & Partner Patentanwälte PartG mbB
(86) International application number: PCT/JP2012/064729
(87) International publication number: WO 2012/165660

(57) **Abstract**

With an ultrasonograph, medical information of a heart could not be accurately obtained as the cardiac apex of the heart could not be recorded clearly. A biomedical information measuring device capable of obtaining accurate medical information is created by measuring an apexcardiogram in M-mode using a linear probe, and improving a data processing device so as to establish an algorithm useful in accurate evaluation so as to solve the above problem.

## Description

### TECHNICAL FIELD

The present invention relates to a probe capable of grasping motions of the heart of a living body, and a biomedical information measuring device and a biomedical information measuring method capable of grasping medical information of the living body through grasping the motions of the heart of the living body. More specifically, it relates to a biomedical information measuring device and a biomedical information measuring system capable of measuring an apexcardiogram using an ultrasound probe of an ultrasonograph and accurately displaying medical information of the living body, and a biomedical information measuring method used by the device and the system.

### BACKGROUND ART

Currently, cardiovascular diseases keep increasing and there is worldwide desire to overcome them. There are many examples of cardiovascular diseases such as congenital heart disease, hypertension, hypertensive heart disease, acute myocardial infarction, old myocardial infarction, hypertrophic cardiomyopathy, dilated cardiomyopathy, secondary cardiomyopathy, aortic stenosis, aortic regurgitation, mitral stenosis, mitral regurgitation, arteriosclerosis obliterans, chronic heart failure and arrhythmia for which improvement in medical technology and preventative measures is desired.

A measuring device capable of objectively grasping motions of the heart allows fairly accurate understanding of the health condition of a living body to be measured and may be useful in diagnosis and therapy. For example, the health condition of a person is greatly dependent on motions of the heart. A medical specialist having advanced cardiovascular knowledge may understand pathophysiology through palpation by placing his/her hand on the chest wall of a patient.

A means of objectively expressing information of motions of a heart obtained through palpation is an apexcardiogram. Use of a measuring device capable of measuring an apexcardiogram of a living body to be measured, including healthy persons as well as patients having a disease of the heart and being limited in motion thereby, without causing particular stress on the living body to be measured at bedside in a normal room rather than in a soundproof room can bring great news for health management and treatment of the living body to be measured.

However, a practical measuring device capable of accurately measuring an apexcardiogram of a person as the living body to be measured has unfortunately not yet been developed. Use of a mechanocardiogram recording device described in "Practice of measuring about electrocardiogram and mechanocardiogram" of Non-patent Document 1 has been attempted; however, as described later, it is branded as a device that cannot be simply used for individual health management even at the medical front.

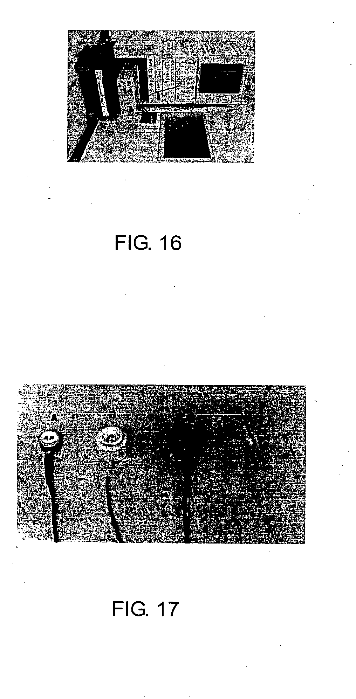

FIG. 16 illustrates the conventional mechanocardiogram recording device described in Non-patent Document 1, which is costly, large with a width of approximately 60 cm, height of approximately 180 cm, and depth of approximately 80cm, and heavy enough that it is difficult for a single person to move even though it has a trolley. At least one measuring technician is required inside and outside a soundproof room respectively, and in many cases, measuring is carried out by at least a medical doctor and a technologist. In the drawing, a window found on the right side of the mechanocardiogram recording device is for peeking inside the soundproof room in which the living body to be measured is placed. FIG. 17 illustrates a photograph of various exemplary transducers described on page 212 of Non-patent Document 1.

Since the device of FIG. 16, when measuring an apexcardiogram, cannot measure without having the living body to be measured enter a soundproof room or a measuring room with little noise, and must measure in a room that has limitations such as being soundproof and a closed chamber, there are problems such as the living body to be measured is measured under abnormal conditions and is thereby under unnecessary stress, the living body to be measured is limited to a person in a condition capable of enduring measurement in such an environment, and measurement of patients for which diagnosis using the apexcardiogram is truly necessary is impossible. Moreover, the current status is that there are many cases where, even in the opinion of a medical specialist having advanced knowledge in the field, the aforementioned cardiovascular disease cannot be diagnosed with certainty from the apexcardiogram measured using the mechanocardiogram recording device of FIG. 16.

In recent years, the ultrasonograph has made great strides, has been widely used at the medical front, and can observe motions of each cardiac chamber within the heart, size of each cardiac chamber, thickness of walls of ventricles and atria, condition of pericardial fluid, motions of valves, blood circulation, etc. from outside of the chest wall, and cardiovascular specialists have been diagnosing cardiovascular patients by looking at those results. As described above, it is assumed that information required for diagnosis cannot be obtained from an apexcardiogram measured using the conventional mechanocardiogram recording device, and that it is better to diagnose by obtaining various kinds of information using an ultrasonograph rather than an apexcardiogram measured using the conventional mechanocardiogram recording device.

However, the ultrasonograph is not made with the objective of measuring an apexcardiogram. It captures motions of the heart, motions of valves, blood flow, and the like as moving images, and a medical specialist looks at those images and diagnoses whether it is normal or pathological. From this measurement, information from which can be determined motions of ventricles, normal/abnormal of the aortic valve and the mitral valve, degree of stenosis, valve area, existence of bacterial vegetation adhered to valves, existence of pericardial effusion, existence of reflux of blood and degree thereof, and existence of a congenital anomaly can be obtained. However, motions of the left ventricular apex are not evaluated via the chest wall, and such information that is obtained through palpation cannot be objectified. That is, since a working measuring device has not been available until now, measured data of the apexcardiogram that is useful in diagnosis is insufficient, technology for diagnosing cardiovascular diseases from the measured data of the apexcardiogram is not established, and therefore cannot be used at the medical front. For that reason, the only measuring device capable of objectively displaying motions of the left ventricle (also referred hereafter as LV) of a heart obtained through palpation are those devices described in Patent Documents 1 and 2 proposed by the present inventor, although application thereof is still not established.

### Prior Art Documents

### Patent Documents

Patent Document 1: JP 2008-113728A
Patent Document 2: JP 2009-273817A

### Non-patent Documents

Non-patent Document 1: "Practice of measuring about electrocardiogram and mechanocardiogram", published by Japanese Association of Medical Technologists (aggregate corporation), second printing Nov. 1, 1996, pgs 212, 222
Non-patent Document 2: "Gray's Atlas of Anatomy", published by Elsevier Japan KK, Sept. 15, 2008, e.g., pg 87

### DISCLOSURE OF THE INVENTION

### Problem to be Solved by the Invention

With the ultrasonograph currently in practical use, even if a medical specialist takes images of a heart thought to have an abnormality, it is currently extremely difficult to diagnose health condition, particularly a pathological health condition of a living body from those images because the cardiac apex images of the heart of respective images are blurred.

The present invention has been devised in light of such situation, and an object of the present invention is to provide at low cost an ultrasonograph having functions of measuring, evaluating, and displaying an apexcardiogram.

An object of the present invention is to provide a measuring device at a low cost based on new technical ideas combining technical ideas of measurement by echocardiography available at the medical front and technical ideas of apexcardiogram measurement, provide a measuring device and a data processing algorithm thereof available at the medical front without measuring and researching an enormous number of living bodies to be measured for a device user who has installed the device, and provide a biomedical information measuring method applicable to a biomedical information measuring device and a biomedical information measuring system that are user friendly at the medical front and capable of accurately grasping health condition of a living body, and a probe for use therewith.

Moreover, an object of the present invention is to provide a biomedical information measuring method applicable to a biomedical information measuring device and a biomedical information measuring system capable of more accurately obtaining biomedical information of the left ventricular function by combining a biomedical information collecting function obtained using a conventional ultrasonograph and a biomedical information collecting function obtained using an apexcardiogram measuring device so as to make it more applicable.

Furthermore, an object of the present invention is, from the perspective of medical education, to provide a biomedical information measuring device, a biomedical information measuring system and a biomedical information measuring method capable of highly accurate measurement at a low cost that allows great improvement in clinical medical education such that proper physical examination may be instructed to medical students and medical residents.

### Means of Solving the Problem

The problem has been solved by measuring an apexcardiogram utilizing the ultrasonograph that could not be created with the purpose of recording an apexcardiogram conventionally, creating an evaluation algorithm thereof, and displaying the apexcardiogram including evaluation results on a display screen.

As a preferred embodiment, the problem has been solved by internally incorporating or externally mounting in the ultrasonograph a program for displaying a measured apexcardiogram as a line drawing and an algorithm for evaluating the apexcardiogram.

The problem has also been solved by displaying an apexcardiogram and other echocardiograms in turn on a display screen or by displaying them simultaneously on the same screen.

Moreover, probes such as linear probes and sector probes, are proposed where the probes are integrated in the lengthwise direction in a measurement frame having guide rails, for example, appropriate probe for measuring is slid and moved along the rails to a portion coming into contact with a body surface in the frame so as to measure an apexcardiogram without changing over or with a minimal change-over operation, and a two-dimensional echocardiogram, for example, an apical long axis four chamber view may be measured. Furthermore, integration of probes mutually differing in frequency is also effective.

### Results of the Invention

According to the biomedical information measuring device, the biomedical information measuring system and the biomedical information measuring method applicable thereto of the present invention, great results are brought about, such as it is possible to measure an apexcardiogram at bedside or even in daily life and apply diagnosis information obtained from the apexcardiogram together with diagnosis information of a living body to be measured obtained using the conventional ultrasonograph, and obtain medically accurate medical information of the heart of the living body to be measured.

Moreover, the present invention provides a measuring apparatus allowing a user of a biomedical information measuring device to obtain medical information to a certain degree. It also exhibits extremely great results of being able to establish a new prospective health management system capable of inputting measured information to a measuring apparatus main unit and carrying out health management of many living bodies remotely or collectively by joining a measuring apparatus terminal and the measuring apparatus main unit by a radio communications system, for example, or applying the measuring apparatus terminal to an information input portion for the measuring apparatus main unit, and capable of developing social health management.

### BRIEF DESCRIPTION OF DRAWINGS

FIG. 1 is an explanatory diagram of a biomedical information measuring device according to an embodiment of the present invention;
FIG. 2 is a diagram describing a display screen of the present invention;
FIG. 3 is a diagram describing a display screen of the present invention;
FIG. 4 is a diagram describing a display screen of the present invention;
FIG. 5 is a schematic diagram describing a living body to be measured according to the present invention;
FIG. 6 is a schematic diagram describing motions of each part in a living body in a tomographic image including an ultrasound probe 11, the cardiac apex, and/or a portion between the vicinity thereof;
FIG. 7 is a schematic diagram describing motions of the heart in the endsystolic phase;
FIG. 8 is a schematic diagram describing motions of the heart in the diastolic phase;
FIG. 9 is a diagram describing a measuring position for an apexcardiogram in the biomedical information measuring device as an embodiment of the present invention;
FIG. 10 illustrates an exemplary apexcardiogram measured utilizing ultrasound waves as an embodiment of the present invention;
FIG. 11 illustrates an exemplary apexcardiogram measured utilizing ultrasound waves as an embodiment of the present invention;
FIG. 12 illustrates an exemplary apexcardiogram measured using the biomedical information measuring device as an embodiment of the present invention;
FIG. 13 illustrates an exemplary apexcardiogram measured using the biomedical information measuring device as an embodiment of the present invention;
FIG. 14 illustrates an exemplary apexcardiogram measured utilizing ultrasound waves as an embodiment of the present invention;
FIG. 15 illustrates an exemplary apexcardiogram measured utilizing ultrasound waves as an embodiment of the present invention;
FIG. 16 shows a photograph of a conventional mechanocardiogram recording device; and
FIG. 17 shows a photograph of various exemplary conventional transducers.

### Description of Reference Numerals

11: ultrasound probe
12: body surface
14a: fibrous pericardum
14b: epicardium
15: myocardium
16: endocardium
18, 43: phonocardiogram
34: right ventricle
40: left ventricle
44: apexcardiogram
45: electrocardiogram
300: biomedical information measuring device
301: ultrasound probe
305a: phonocardiogram-sensor
305b: electrocardiogram (ECG)-sensor
320: control unit and measured data processing unit
330: memory unit
340: display unit
F1, F11∼F13, F21∼F23, F31∼F33, F41, F42: display screen
P(1) ∼ P(7): characteristic points of apexcardiogram
R: wave R of ECG

### DESCRIPTION OF EMBODIMENTS

Embodiments of the present invention are explained with reference to drawings hereafter. Note that each drawing used for explanation is schematically illustrated to such an extent that the embodiments of the present invention are understandable. Moreover, description of the biomedical information measuring method may serve as that of the biomedical information measuring device without particular notice to the effect, or vice versa, within the scope of being understandable and without any misunderstanding to those skilled in the art.

It is difficult to obtain clear images from those taken using the ultrasonograph as the cardiac apex is too close to the body surface. However, the inventor has tried various measurements with a view to improve the device from the perspective that if motions of the cardiac apex are recorded as some form of data, shortcomings of the current ultrasonograph should be compensated. As a result, even if accurate diagnosis is impossible with the conventional images due to unclearness, if the apexcardiogram can be measured and an evaluation algorithm capable of obtaining medical information therefrom can be attained, it is found to be useful in compensating the shortcomings of the current ultrasonograph, thereby leading to completion of the present invention. Details thereof are explained with reference to drawings hereafter.

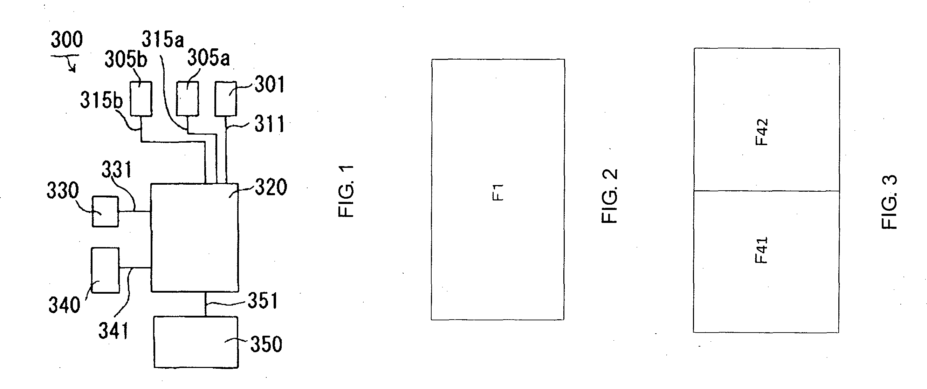

FIG. 1 is a block diagram describing a biomedical information measuring device as an embodiment of the present invention. This is a biomedical information measuring device that can be remotely managed. In FIG. 1, reference numeral 300 denotes the biomedical information measuring device according to the embodiment of the present invention, and 301 denotes an ultrasound probe as a sensor of an apexcardiogram for measuring the apex beat, which includes an ultrasound transmitter having multiple ultrasound transmitting elements or ultrasound transducers and a receiving sensor having multiple reflected signal receiving elements or reflected signal receivers for receiving a signal that has entered a living body and reflected off of each part in the living body. Reference numeral 305a denotes a phonocardiogram-sensor for measuring heart sounds; 305b denotes a sensor such as an ECG-sensor for measuring an ECG; 320 denotes a control unit and a measured data processing unit, 330 denotes a memory unit, 340 denotes a display unit, 350 denotes a remote management unit, 311 denotes a connecting means connecting between the ultrasound probe 301 and the control unit and the measured data processing unit 320, 315a denotes a connecting means connecting between the sensor 305a and the control unit and the measured data processing unit 320, 315b denotes a connecting means connecting between the sensor 305b and the control unit and the measured data processing unit 320, 331 denotes a connecting means connecting between the memory unit 330 and the control unit and the measured data processing unit 320, 341 denotes a connecting means connecting between the display unit 340 and the control unit and the measured data processing unit 320, and 351 denotes a connecting means connecting between the remote management unit 350 and the control unit and the measured data processing unit 320. At least a part of the connecting means may be constituted using a communication means such as wireless communication or optical communication.

Note that the biomedical information measuring device 300 may constitute a biomedical information measuring system combined with several devices each having the respective aforementioned functions. The phonocardiogram-sensor 305a is a sensor for measuring heart sounds and the ECG-sensor 305b is a sensor for measuring an ECG, neither of which need to be built into the main device.

In FIG. 1, the ultrasound probe 301, the phonocardiogram-sensor 305a and the ECG-sensor 305b are respectively placed on a measuring place of the living body to be measured, signals for measuring an apex beat, heart sounds and an ECG are detected, respectively, the detected signals are input to the control unit and the measured data processing unit 320 via the respective connecting means, and data processing is conducted as an apexcardiogram, a phonocardiogram, and an ECG. As a preferred example, apexcardiogram data and phonocardiogram data are extracted in sync with the ECG signal, and then ECG data, the apexcardiogram data, and the phonocardiogram data are stored and displayed as a graph. Moreover, input of a standard waveform pattern from the outside and use thereof in classification of waveforms of the apexcardiogram allows further accurate evaluation according to the living body to be measured, for example.

Data from the sensors is processed as data for creating an apexcardiogram and other data, stored in the memory unit, and displayed on the display unit as needed. The aforementioned other data, which may be unnecessary, may be, for example, two-dimensional distribution of reflection intensity within a predetermined range of the chest wall, contact pressure, etc. Economically, a conventional linear sensor, for example, is available as the ultrasound probe.

Regarding measurement of the apexcardiogram according to the present invention, as a preferred embodiment, an apexcardiogram is measured by providing an ultrasound transmitting element and an ultrasound receiving element in ultrasound probes, and a signal resulting from an ultrasound wave transmitted from an oscillating element that hits the cardiac apex and/or the vicinity thereof and is reflected therefrom is received by the receiving element. To give an example, an apexcardiogram is measured using 192 ultrasound transducers, wherein each of twelve-transducer groups transmits to scan in turn one after another, and a time is prepared for the same element to function as an oscillating element and to function as a receiving element through timing control so as to receive the reflected ultrasound wave from the cardiac apex and/or the vicinity thereof.

Alternatively, as another embodiment of the present invention, an ultrasound transmitting element and an ultrasound receiving element are provided as ultrasound probes, the ultrasound transmitting element transmits an ultrasound wave of a single kind of wavelength from a single oscillating element, and the transmitted ultrasound wave from the same oscillating element that has hit the cardiac apex and/or the vicinity thereof and reflected therefrom is received by the ultrasound receiving element having a receiver or made of multiple receiving elements arranged adjacently and capable of independently receive waves so as to measure an apexcardiogram. In the case of using multiple kinds of ultrasound waves as pulsation-measuring signals, it is preferable to use an ultrasound oscillating element for each kind. Multiple kinds of ultrasound frequencies may also be used.

In the case of transmitting and receiving ultrasound receiver signals from the ultrasound probe, each of twelve-transducer groups transmits to scan in turn one after another, for example, a more accurate graph may be made by displaying a receiver signal from each receiving sensor element at respective times in time series, and supplementing long reception time intervals through interpolation. It is preferable that a method of electronically scanning by the probe is changeable according to purpose, for example.

A biomedical information measuring device or a biomedical information measuring system that can be remotely processed may be configured by providing at least a part of contents of the control unit and the measured data processing unit 320, the memory unit 330, and the display unit 340 for the remote management unit 350 in FIG. 1.

Furthermore, as a preferred embodiment of the present invention, while not being limited narrowly to this example, multiple ultrasound transmitting elements and multiple ultrasound receiving elements may be arranged in a line or vertically and horizontally in the ultrasound probe 301, and the so-called ultrasound probe used for measuring an ultrasonic echo may be used by scanning each element. As an example of the arrangement method of the ultrasound transmitting element and the ultrasound receiving element, a plurality of the elements may be arranged one-dimensionally or two-dimensionally. Arrangement of either method has confirmed that the apexcardiogram of the present invention can be measured.

In the case of providing an acoustic lens at an end part of the ultrasound probe, namely a portion to touch the body surface, silicone rubber or a liquid substance, for example, may be used as a medium acting as a cushion or the acoustic lens. Pressure may be applied on this medium so as to vary density and refractive index, and definition, for example, may be adjusted.

Use of a two-dimensional arrangement of ultrasound transducers on the ultrasound probe 301 allows exhibition of great results of further accurate measurement of motion per pulsation of the cardiac apex, obtainment of important information that clarifies relevance between medical information and motions of the cardiac apex of individual living bodies that has been unclear until now, and assistance in accurate health management.

As an embodiment of the present invention, a highly reliable apexcardiogram may be obtained upon measurement using ultrasound probes, each having 192 ultrasound transducers arranged in a line approximately 0.2 mm apart, arranged two-dimensionally in three lines, namely ultrasound probes having 192 times 3 transducers arranged two-dimensionally. Further increase in number of lines of the transducers allows obtainment of detailed information on motions of the cardiac apex and further accurate medical information.

Alternatively, it is possible to provide one or multiple lines of multiple transducers arranged in a line and provide a sensor for detecting presence of a rib so as to constitute an ultrasound probe, which has a buffering agent of a material with little attenuation of ultrasound waves on a surface touching the body surface of the living body, place the probe on a measuring place of the left chest wall, for example, of the living body to be measured, and detect presence of a rib directly beneath the probe (position within a predetermined distance from the probe) during measurement. In the case where presence of a rib is detected, controlling the transducers arranged in inter-costal space to be in an active state but controlling transducers arranged in the vicinity of the rib within a predetermined range to be in a non-active state and measuring motions of the cardiac apex and the vicinity thereof allow measurement of a further accurate apexcardiogram. Furthermore, diverse changes in control of scanning may be adopted for understanding complex motions of the cardiac apex. Moreover, there is an advantage that by arranging sensor elements with different purposes three-dimensionally and multi-dimensionally in addition to the probes having transducers arranged two-dimensionally, more of biomedical information can be obtained.

Furthermore, the probes, for example linear probes and sector probes, are integrated in the lengthwise direction in a measurement frame having guide rails, for example, appropriate probe for measuring is slid and moved along the rails to the portion coming into contact with the body surface in the frame so as to measure the apexcardiogram without changing over or with a minimal change-over operation, and a two-dimensional echocardiogram, for example, an apex long axis four chamber view may be measured. Making the probes flexible is advantageous in that detection sensitivity can be heightened, etc.

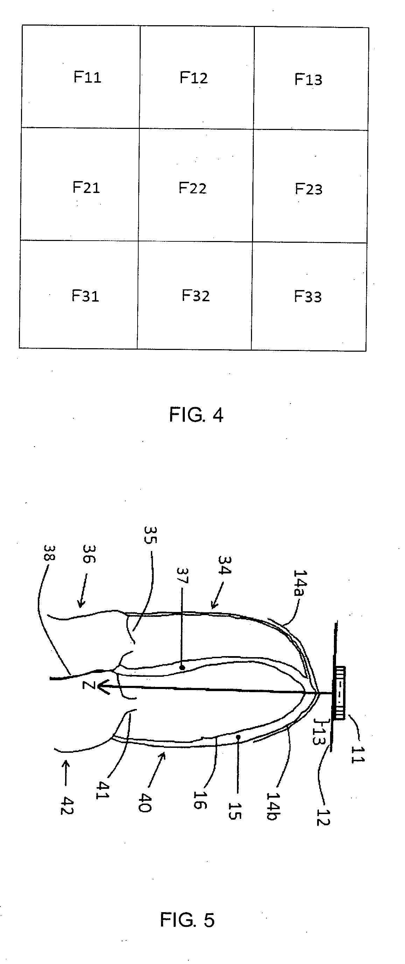

FIGS. 2 to 4 are examples of display screens of the biomedical information measuring device using ultrasonic echo technology according to the present invention. FIG. 2 illustrates a display screen for alternately displaying a two-dimensional echocardiogram, for example, an apical long axis four chamber view and an apexcardiogram on a display screen with display screen reference numeral F1 and then comparing them. FIG. 3 illustrates a display screen for displaying a two-dimensional echocardiogram, for example, an apex long axis four chamber view on a display screen with display screen reference numeral F41 and an apexcardiogram on a display screen with a display screen reference numeral F42 and then comparing them. FIG. 4 illustrates a display screen for displaying an updated apexcardiogram on a display screen with display screen reference numeral F22, displaying various images (for example, apical long axis four chamber view) of a two-dimensional echocardiogram on a display screen with display screen reference numerals F11 to F13, displaying a color Doppler image on a display screen with display screen reference numeral F21, displaying temporal changes of the apexcardiogram on a display screen with display screen reference numerals F23 and F31 to F33, and then comparing changes therebetween. When the measured apexcardiogram is determined or evaluated as abnormal using an algorithm of the present invention described later, automatic recognition of the results thereof may be carried out so as to display ultrasonic echo images other than the apexcardiogram in accordance therewith in the display screens F11 to F13, etc.

Motions of the cardiac apex at each pulsation of a heart differ due to difference in living body to be measured, where those motions are extremely complex and even the same living body to be measured moves differently depending on health conditions. If an extreme change in motions of the heart is discovered by looking at a moving or still image during echo sounding using the conventional ultrasonograph, while it is sensed that something is wrong, it is often difficult to make a diagnosis just from that alone, even if it is a very remarkable change. There are not many cases where state of disease of a living body to be measured can be diagnosed just from this observation alone, where diagnosis is made in conjunction with other diagnostic information.

However, motions of the cardiac apex are transmitted to the palm during palpation. Data or information superior to palpation findings can be obtained when a plurality of the transducers is arranged two-dimensionally and the data is obtained across a plurality of inter-costal spaces. It is found that very important information can be obtained if 100 or more, preferably 150 or more transducers are arranged in parallel in one direction along the probes within visual field width of the probes of 30 by 20 mm, preferably 30 by 30 mm. However, a solution to the problem that a shadow, noise, etc. due to presence of a rib hinders increase in high accuracy of the apexcardiogram is in demand.

As a result of research, this problem can be improved significantly even by changing the ultrasound transmitting elements and the receiving elements for measurement of the apexcardiogram in the vicinity of the position of the rib, for example, directly above the rib to non-active states.

Great results such as being able to reduce noise during measurement so as to obtain a clearer apexcardiogram by making the ultrasound signal for detecting the position of the rib to a different signal than the ultrasound signal for measuring the apexcardiogram, for example, a signal with a different frequency or a modulated signal may be achieved. It is preferable to make the ultrasound signal for detecting the position of the rib be from 10 to 15 MHz, preferably 12 MHz. In light of manufacturer's cost, it is preferable to set frequency of the ultrasound signal for detecting the position of the rib to a harmonic component such as a second order harmonic of an ultrasound frequency for measuring the apexcardiogram.

Upon measurement of an apexcardiogram using ultrasound signals of various frequencies, the inventor found frequencies of 10 MHz or less to be preferable, and that definition of the apexcardiogram according to the present invention decreased at higher frequencies. Frequencies of 3 MHz or greater are preferable, and 5 to 8.5 MHz is further preferable. 7.5 MHz is preferred in light of cost.

According to purpose of measurement, it is preferable to combine a plurality of fundamental sensor parts with an elongated cross-sectional form, like a fundamental transducer arranged plate having at least either component parts where a plurality of the transducers are arranged one-dimensionally or two-dimensionally in a direction orthogonal to the transmitting direction of the ultrasound, so as to constitute ultrasound transmitters and receiving sensors of the ultrasound probes. The plurality of transducers constituting the ultrasound transmitters may also act as the plurality of transducers constituting the receiving sensors, so as to be used in measurement through electronic scanning in compliance with a predetermined sequence.

Moreover, sensors for different measurement purposes from measurement of an apexcardiogram itself, such as a sensor for measurement of contact pressure, may be arranged at staggered positions in the ultrasound transmitting direction of the probes, that is, transducers and pressure sensors may be arranged three-dimensionally, thereby heightening measurement results.

A structure where an elastic body with little attenuation of ultrasound waves is placed between edges of the transducers of the ultrasound probes and outer wall of the body surface of the living body to be measured is preferred. This elastic body may be constituted by low cost silicone rubber. Moreover, this elastic body may be constituted by a liquid, or it may be a sol-like material, a gelatinous material, or a colloidal matter. In the embodiments of the present invention, it is preferable to give this elastic body a function of an acoustic lens. Making refractive index of the acoustic lens be variable entirely or partially allows increase in measurement accuracy. The refractive index may be changed partially or entirely by applying pressure on the elastic body, for example.

Measured data detected by the two-dimensionally arranged sensors or multi-dimensionally arranged sensors is stored as digital data in the memory unit, and appropriate data for various analysis purposes may be created by the data processing unit using software installed as needed after measuring.

However, it is confirmed that even if the ultrasound probe 301 in which the ultrasound transducers are arranged one-dimensionally is used, the apexcardiogram of the present invention can be measured and an apexcardiogram can be measured using probes with a visual field width of approximately 38 mm, within which 192 ultrasound transducers, for example, are each arranged one-dimensionally at intervals of approximately 0.2 mm. Provision of an apexcardiogram creating means to the ultrasonograph may configure the biomedical information measuring device and the biomedical information measuring system of the present invention.

Alternatively, as a preferred embodiment of the present invention, while not being limited narrowly to this example, utilization effect of the apexcardiogram measured according to the present invention may be heightened. For example, it is possible for the ultrasound probe 301 to be mounted with a power component or component equivalent to a power source for driving a sensor or the like or a communication part, constitute the ultrasound probe 301 in a state not wire-connected to the biomedical information measuring device main unit, transmit the measured data of the ultrasound probe 301 to the control unit and the measured data processing unit 320 provided in the biomedical information measuring device main unit using a transmitting means such as radio waves or light (referred to as light that includes infrared light and ultraviolet light), measure an apexcardiogram with high quality, and carry out a wider range of health management.

With the present invention, when such an ultrasound probe, which is adhered to the living body to be measured, has a part with an important function of detecting the apex beat, this part is also a biomedical information measuring device of the present invention or a part for the biomedical information measuring device. Note that the ECG-sensor and the phonocardiogram-sensor may be given a data acquiring function and a communication function, respectively, and used independently from the biomedical information measuring device main unit, or they may be wire-connected to the biomedical information measuring device main unit.

Measuring conditions are as follows. Basically, measurement is taken either in the left lateral decubitus position or the semi-lateral left decubitus position. While measuring is preferably performed in a semi-expiratory phase in a relaxed state, appropriate measurement is taken in accordance with condition of the living body to be measured. With a method of measuring without stopped breathing during respiration at rest, a base line of the apexcardiogram shakes, making it difficult to evaluate. When measuring in an end expiratory phase (point when breath is completely expelled), since it is easy to have heightened tension of muscles and therefore to enter a state of breath holding, evaluation may be difficult. It is known that breath holding effects change in blood pressure and heart rate, and since it is in a different state than at rest, unfavorable conditions may result in the end-expiratory phase. Accordingly, much of the data given below is, as a general rule, data resulting from measuring the body in the left lateral decubitus position or the semi-lateral left decubitus position for a predetermined period in a relaxed state while lightly holding his/her breathing in a semi-expiratory phase.

While the aforementioned measuring conditions are particularly desirable conditions, with the present invention, it is understood that an apexcardiogram can be measured effectively even when breathing, and medical information of the living body to be measured can be detected.

In such a measurement, an apexcardiogram of the living body to be measured is measured using the biomedical information measuring device of the present invention either at an outpatient clinic or a general hospital room. Electronic leads for ECG are attached to the right upper limb and the left lower limb of the living body to be measured as a general rule, an ECG is measured using lead II of a standard 12 leads electrocardiogram, and a phonocardiograph microphone is attached to the mid left sternal border as a general rule. The living body to be measured is then placed in the left lateral decubitus position or the semi-lateral left decubitus position, a measuring technician touches the apex beat and confirms a region with the strongest pulsation, and an ultrasound probe is placed against that region as a pulsation sensor so as to measure an apexcardiogram. All of the above operations, measurements and recordings can be easily performed by a single measuring person. Preparations for measuring an ECG, a phonocardiogram and an apexcardiogram are made and measuring is started.

According to a preferred embodiment of the present invention, to begin with, measurement data is taken in sync with a wave R of an electrocardiogram (ECG) waveform (QRS complex). The measurement data includes, for example, at least three items of the ECG, the phonocardiogram and the apexcardiogram. Information of the ECG and the phonocardiogram is useful in analysis of the apexcardiogram. For example, identification of the wave R of the ECG is required, which can be found by a well-known method. Information of a first heart sound, a second heart sound, a third heart sound, a fourth heart sound, and so on of a phonocardiogram and information of carotid pulse waves may be useful. Identiflcations of these are also found through a well-known method.

Moreover, application of measurement information such as the B mode method, the Doppler method, etc. for the conventional ultrasonograph may be useful in analysis of the apexcardiogram. It is the first time with the present invention that use of a plurality of pieces of measurement information in real time in particular is possible, leading to great advances in diagnosis of the cardiac function.

Sensors for the ECG and the phonocardiogram in addition to the ultrasound probe may also be arranged in the sensor for the apexcardiogram. In this case, remarkable results such as considerable simplification of operations may be achieved. Use of a temperature gauge or a barometer leads to even greater results.

The inventor of the present invention has succeeded in creating an analyzing means capable of analyzing association between the apexcardiogram and pathophysiology with extremely high accuracy as a result of detailed study of measurement examples of many living bodies to be measured using the biomedical information measuring device of the present invention. Upon comparison of the analysis results according to the present invention with information of diagnoses made through other means such as MRI, myocardial scintigraphy, echocardiography, cardiac catheterization, etc., information obtained through inspection, palpation and auscultation, and therapy and medical information of the living bodies to be measured, medical information of the living bodies to be measured obtained using the present invention is found to be extremely reliable.

FIG. 5 is a schematic diagram describing measurement of an apexcardiogram by placing an ultrasound probe of a biomedical information measuring device, as an embodiment of the present invention, on the body surface of a living body to be measured. Reference numeral 11 denotes an ultrasound probe, 12 denotes a body surface of a living body to be measured, 13 denotes skin and skeletal muscle, 14a denotes the fibrous pericardium, 14b denotes the epicardium, 15 denotes the myocardium, 16 denotes the endocardium, 34 denotes the right ventricle of the heart (also referred to as right ventricle or RV), 35 denotes the tricuspid valve, 36 denotes the right atrium, 37 denotes the interventricular septum, 38 denotes the interatriar septum, 40 denotes the left ventricle, 41 denotes the mitral valve, 42 denotes the left atrium, and Z denotes a Z-axis. An X-axis and a Y-axis orthogonal to each other within a plane orthogonal to the Z-axis are defined.

In a preferred embodiment of the present invention, it is preferable to measure an apexcardiogram in the left lateral decubitus position or the semi-lateral left decubitus position in the case of a living body to be measured in the normal cardiac position having the heart on the left side. In FIG. 5 of this case, the body surface 12 on which the ultrasound probe 11 is placed is the left chest wall of the living body to be measured. Moreover, in the case of the living body to be measured having dextrocardia where the heart is slightly to the right of the mid portion, it is preferable to measure an apexcardiogram in the right lateral decubitus position or the semi-lateral right decubitus position. In FIG. 5 of this case, the body surface 12 on which the ultrasound probe 11 is placed is the right chest wall of the living body to be measured.

In a preferred embodiment of the present invention, it is preferable to measure an apexcardiogram using a probe with a long and thin end as the ultrasound probe 11, and although omitted from FIG. 5, arranging the probe in an intercostal space (between adjacent ribs) where it is most sensitive with the longitudinal direction of an end of the ultrasound probe 11 set as longitudinal direction of the ribs. This allows obtainment of an apexcardiogram from which health condition of the living body to be measured can be further accurately determined.

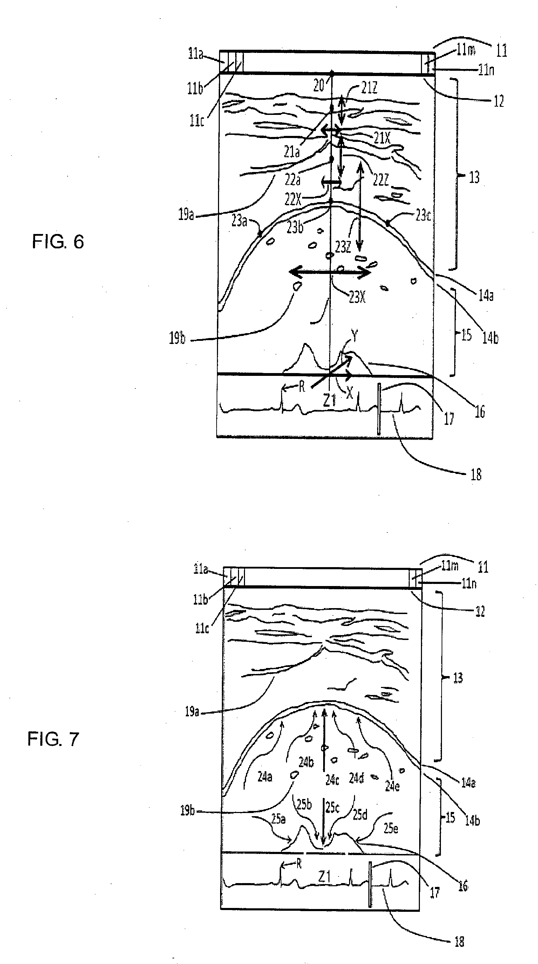

FIG. 6 is a schematic diagram describing measurement using the biomedical information measuring device as an embodiment of the present invention and motion of each part in a living body in a tomographic image including the ultrasound probe 11, the cardiac apex and/or a portion between the vicinity thereof by placing the ultrasound probe 11 on the body surface 12 of the left chest wall in the semi-lateral left decubitus position of a person in the normal cardiac position. Reference numerals 11a∼c, ...11m, and 11n respectively denoting a transducer constituting the ultrasound probe 11 are arranged in parallel with each other, and may also be used as transmitting elements and receiving elements for ultrasound signals through electronic scanning. 17 denotes a mark indicating a time phase on the ECG, representing a time phase in the illustrated schematic diagram. 18 denotes an ECG, 19a denotes fibrous tissue within the skin and the skeletal muscle 13, and 19b denotes fibrous tissue in the myocardium 15. 20 denotes a point on the body surface on a Z1-axis as the Z-axis; 21 a denotes a point relatively near the body surface on the Z1-axis; 22a denotes a point in the skin and skeletal muscle 13 at a deeper position than the point 21 a, namely at a position near the heart; 23b denotes a point above the fibrous pericardum 14a on the Z1-axis; 23a denotes a point at a position slightly toward the outer side of the left ventricular apex above the fibrous pericardum 14a; 23c denotes a point at a position slightly toward the outer side of the left ventricular apex on the other side of the point 23a above the fibrous pericardum 14a; X denotes an X-axis; and Y denotes a Y-axis. 21X and 21Z denote arrows representing amount of displacement along the X-axis and amount of displacement along the Z-axis of a pulsation at the point 21a, respectively; 22X and 22Z denote arrows representing amount of displacement along the X-axis and amount of displacement along the Z-axis of a pulsation at the point 22a, respectively; and 23X and 23Z denote arrows representing amount of displacement along the X-axis and amount of displacement along the Z-axis of a pulsation at the point 23b, respectively, where longer arrows represent greater amount of displacement.

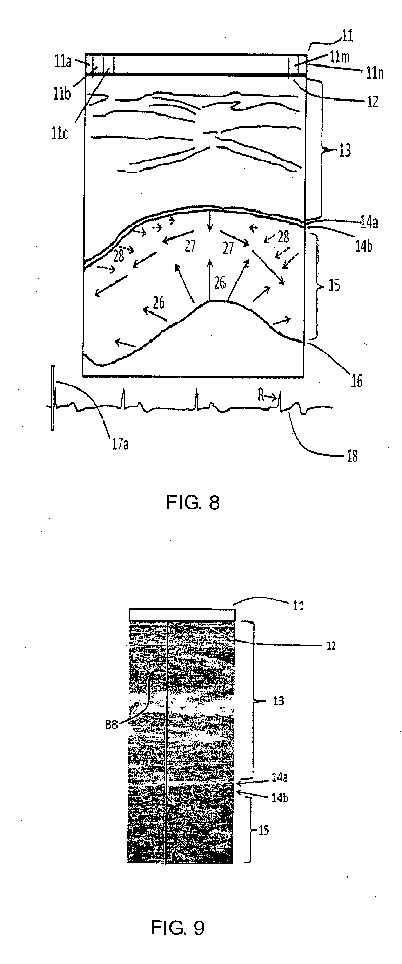

In FIG. 6, ultrasound beams are transmitted to the cardiac apex and/or the vicinity thereof from the body surface 12 of the living body to be measured from the respective transducers 11 a, 11 b, 11 c, ... 11 m, and 11n constituting the ultrasound probe 11. The ultrasound beams that have entered the living body are reflected at different reflection intensities according to difference in reflecting property at the respective parts in the living body and received according to reception mode of the ultrasound probe 11. The data is processed, and while not being limited narrowly to this example, is then displayed on a display screen at brightness proportional to the reflection intensity, for example. In black and white grayscale pictures given in FIGS. 9 to 15 described later, white portions represent portions of strong received reflected signals, black portions represent weak reflected signals, and as is evident from the figures, for example, the centers of white lines are portions where reflection intensity is extremely high. In the schematic diagram of FIG. 6, although not everything is given a reference numeral, portions indicated by reference numerals 19a and 19b, for example, are portions with extremely high reflection intensity. The points 21a, 22a and 23a to 23c are points plotted on the portions with extremely high reflection intensity on the Z1-axis.

The embodiments of the present invention include a means for measuring the amounts of displacement indicated by 21X and 21Z of the portion with extremely high reflection intensity within a predetermined range, for example, detecting change in the amounts of displacement along the Z1-axis, for example, detecting a position at which tendency for amount of displacement to change is great, and detecting position of the fibrous pericardium 14a.

The epicardium 14b, which is directly below the fibrous pericardium 14a, is a membrane attached directly to the heart itself, and the heart itself deflects greatly along not only the Z-axis but also the X-axis and the Y-axis. This contributes to identifying the fibrous pericardium 14a.

Central rays of the respective ultrasound beams that are incident on the cardiac apex and/or in the vicinity thereof via the body surface 12 of the living body to be measured from the respective transducers 11 a, 11 b, 11 c, ... 11 m, 11 n of the ultrasound probe 11 are in parallel to each other. This allows measurement of a practical apexcardiogram of an ultrasonic echo.

FIG. 7 is a schematic diagram describing motions of the heart in the endsystolic phase and is at an ending point of a wave T of the ECG 22, as is indicated by the state of the endocardium 16. The figure shows an example where motions (arrows 24c and 25c) along the Z-axis in FIG. 6 are maximum at the cardiac apex, and illustrates that the respective regions of the left ventricular muscles move in directions indicated by arrows 24a to 24e and 25a to 25e, namely direction of the fibrous pericardium 14a and direction of the endocardium 16.

FIG. 8 is a schematic diagram describing motions of the heart in the diastolic phase. This example shows that motions of respective parts of the myocardium are in directions of arrows 26 and 27 in the early diastolic phase, and in directions of arrows 28 in the end diastolic phase. Motions in the end diastolic phase differ from motions in the early diastolic phase and express motions in opposite directions to those in the early diastolic phase. In normal myocardium, the cardiac apex moves along the body surface in the systolic phase. The cardiac apex in the diastolic phase approaches the body surface just before the second heart sound and then draws away from the body surface. The left ventricular muscles make trembling motions in the early diastolic phase. These motions are pronounced at distal portions from the cardiac apex. It seems that motions of part of the left ventricular free wall are greater than those in part of the interventricular septum. They first deflect outwards at either side of the cardiac apex, quickly returning to the inner lower side. This phenomenon is often recognized in young persons but not much in older persons. This motion is like when soft rubber is fully pulled and momentarily distorted (contracted). Viewed from the central portion of the left ventricular cavity, it moves as if trembling parallel to the periphery of the left ventricular muscles rather than moving vertically. The heart contracts as rubber is wrung and is dilated so as to come loose.

Note that motion of the heart often differs due to health condition, age and other individual characteristics of a living body to be measured, and motion of the heart often differs even in healthy persons, for example.

FIG. 9 is a tomogram measured utilizing ultrasound waves and illustrates a B-mode echocardiogram as a diagram describing a measuring position for an apexcardiogram in the biomedical information measuring device according to the embodiment of the present invention. This figure is a tomogram taken by placing an ultrasound probe (ultrasonic probe) against the chest wall of the living body to be measured so as to produce an image of a part of the heart, and shows positions of body surface, skin, skeletal muscles, the epicardium, and the myocardium of each part of the layer. Reference numeral 88 denotes a line including a position for measuring an apexcardiogram. Specification of a position for measuring an apexcardiogram in the tomogram may be carried out by selecting a position on the line 88 using a cursor, selecting a particular range on the line 88, or select a specific point on the line 88. Alternatively, a display screen of the tomogram may be touched using a pointing device, a measurement range may be drawn using the pointing device, or it may be selected using various other methods.

An actual measuring example using the device of the present invention is given below. Measuring conditions include, as a rule, measuring in the left lateral decubitus position or the semi-lateral left decubitus position, and whenever possible, stop breathing for ten seconds.

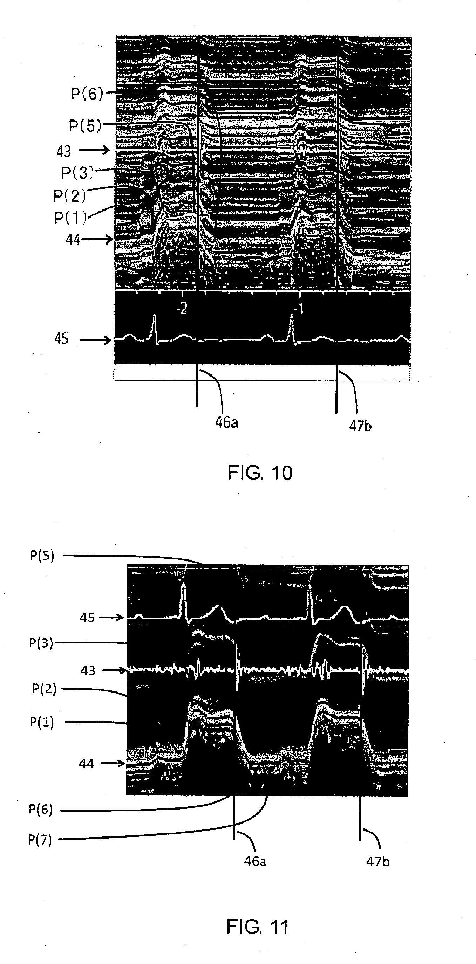

FIG. 10 shows data of a 50-year old male having normal left ventricular systolic function, and illustrates an M-mode echocardiogram as an exemplary apexcardiogram measured utilizing ultrasound waves as an embodiment of the present invention. This apexcardiogram is obtained by applying a probe on the left chest wall of the person in a normal cardiac position, where, as an ultrasound probe, the probe is equipped with an ultrasound transmitter, which scans the plurality of transducers so as to transmit an ultrasound signal to the cardiac apex and the vicinity thereof from the body surface of a living body to be measured, and with a receiving sensor, as an electronic scanning probe, in which a plurality of transducers for measuring reflected waves of the ultrasound waves that entered the living body from each part of the living body to be measured are arranged; transmitting successively ultrasound beams, which are transmitted from the respective transducers, from the body surface to the cardiac apex and the vicinity thereof in accordance with electronic scanning conditions; measuring a B-mode tomogram as described with FIG. 9, selecting a measuring position of the cardiac apex and/or in the vicinity thereof in the tomogram from display screen in B-mode, and measuring temporal change in the reflected ultrasound waves from the measuring position selected in the M-mode. Reference numerals 46a and 47b respectively denote a position of an aortic second sound of the phonocardiogram 43. Characteristic point P(5) described later exists directly before the IIA sounds of phonocardiogram 46a and 47b, and characteristic points P(1), P(2), P(3) and P(6) described later can be confirmed in the same manner. Reference numeral 43 denotes a phonocardiogram, 44 denotes an apexcardiogram, and 45 denotes an ECG. In FIG. 10, the side of the apexcardiogram 44 on which the phonocardiogram 43 is shown is the body surface side and the opposite side thereof is the internal side where the fibrous pericardium exists.

FIG. 11 illustrates an M-mode echocardiogram, which is measured utilizing ultrasound waves as an embodiment of the present invention, and is an apexcardiogram 44 of another living body to be measured having normal left ventricular systolic function. Characteristic points P(1) to P(3) and P(5) to P(7) described later can be recognized clearly.

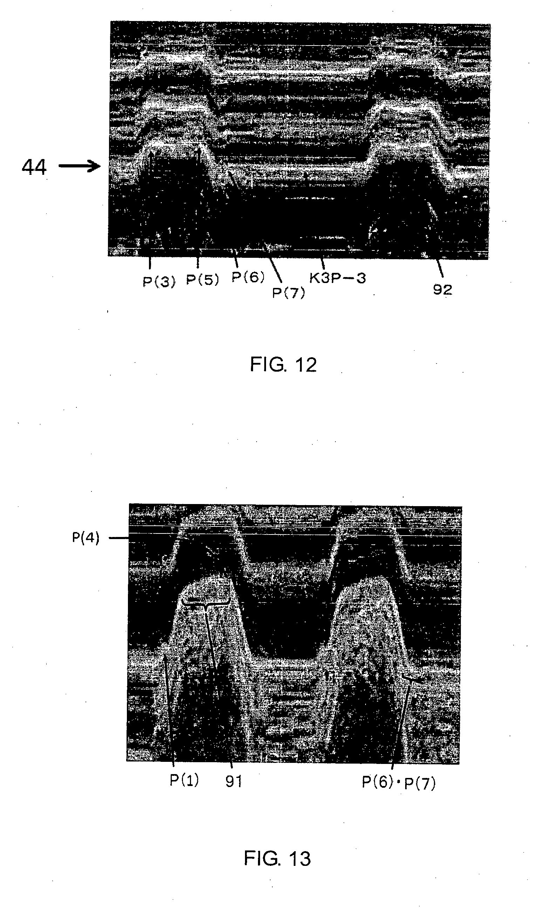

FIG. 12 illustrates an M-mode echocardiogram of a 20-year old male having normal left ventricular systolic function. Reference numeral 44 denotes an apexcardiogram. The characteristic points P(3) and P(5) to P(7) described later can be recognized clearly. Reference numeral K3P-3 denotes an apexcardiogram and 92 denotes a concave portion in the systolic phase.

FIG. 13 illustrates an M-mode echocardiogram of a 60-year old male having left ventricular systolic dysfunction. The P(1) elevates. While there is a rise (reference numeral 91) in the systolic phase, the characteristic point P(4) is recognized but the P(3) and the P(5) described later are not. The characteristic points P(6) and P(7) described later are also recognized.

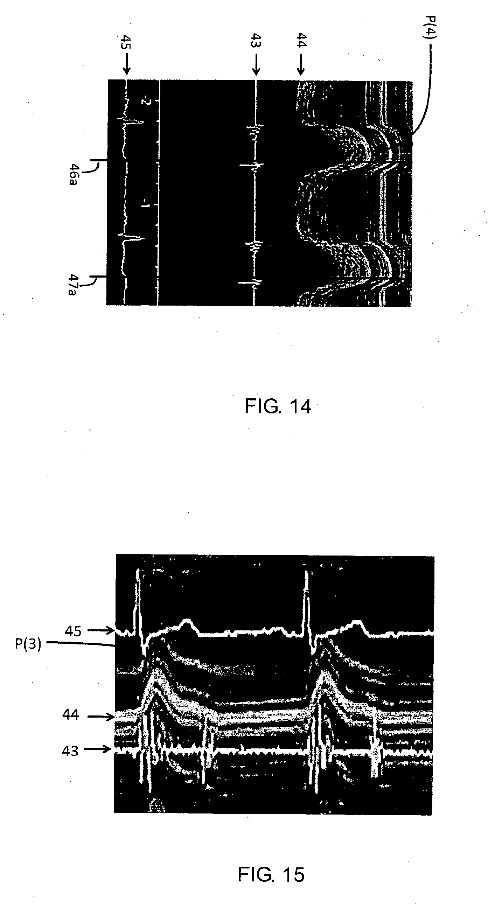

FIG. 14 illustrates an apexcardiogram 44 of an M-mode echocardiogram of another living body to be measured having left ventricular systolic dysfunction, where the characteristic point P(4) is recognized but the P(3) and the P(5) described later are not.

FIG. 15 illustrates an apexcardiogram 44 of an M-mode echocardiogram of a different living body to be measured than that in FIG. 12 having normal left ventricular systolic function, where the characteristic point P(3) described later is recognized but the P(5) and the P(4) are not.

The apexcardiograms of FIGS. 10 to 15 are apexcardiograms respectively made up of the continuous lines where reflection intensity of ultrasound signals is strongest between the fibrous pericardium and the body surface.

Moreover, the apexcardiograms may be made up by accumulating all reflected ultrasound signals between the fibrous pericardium and the body surface in the cross section from respective positions on the body surface to the fibrous pericardium.

Extraction of necessary information for management of a healthy state from this apexcardiogram as biomedical information is important; however, it unfortunately cannot be useful in management of a healthy state unless there is an algorithm applied by appropriately detecting this information. The inventor of the present application has taken many measurements and studied them in detail to find whether this information can be extracted some how and applied, found an algorithm to extract biomedical information, and succeeded in realization of a biomedical information measuring system and a biomedical information measuring device capable of accurately determining health conditions of a living body to be measured from a technical perspective using the algorithm.

The inventor has measured apexcardiograms for many normal left ventricular function persons and abnormal left ventricular function persons using an ultrasonograph, and examined correspondence between the respective characteristic points etc. and medical information. As a result, a conclusion has been drawn that there are many cases where medical information of living bodies to be measured can be accurately estimated.

Types of wave A are classified in the following four groups. Type 1 is a case where wave A is clear, the position P(1) of a positive extreme value is clear, and the P(2) is also clear. Type 2 is a waveform rising from an initial rise of wave A to the P(2). While the P(1) and the P(2) are nearly at the same height, in a first-order differential waveform, a portion corresponding to the P(2) has a negative extreme value (derivative value shifts from negative to positive). Type 3 is a waveform where wave rises continuously until the P(2) but does not have a negative extreme value (derivative value shifts from negative to positive) in the portion corresponding to the P(2) in a first-order differential waveform. The P(1) and the P(2) are defined to be identical in this case. Type 4 is a case where hardly any positive waves are recognized before the P(2), and is thereby not recognized as wave A. When there is no wave A, namely in the case where hardly any positive waves are recognized before the P(2), it is evaluated as not having wave A and the P(1) and point a also does not exist. There are normal cases where there is no load on the end diastolic phase of the left ventricle, and pathological cases where left atrial contractility is lost or decreased.

It is preferable that: when an apexcardiogram waveform of one beat of the living body to be measured is defined as a fundamental waveform, that is, as an example, when the apexcardiogram waveform ranging from a predetermined time before a point (referred to as peak point of the QRS complex hereafter), which corresponds to each positive peak point (R) of respective QRS complex of an ECG at the same time as the apexcardiogram of the same living body to be measured, for example, a lead II ECG in a standard 12 leads electrocardiogram to the predetermined time before a subsequent peak point of the QRS complex is defined as the fundamental waveform, and heart rate compensating coefficient is set to √V/60 (√V/60 means square root of V/60) at a time on a time axis of the apexcardiogram waveform where V denotes heart rate per minute when the fundamental waveform is represented by time on the lateral axis and amplitude of the apexcardiogram waveform on the longitudinal axis; and the time is multiplied by √V/60 and the resulting value is defined as a converted value for the heart rate of 60 on the time axis of the following apexcardiogram; C1 is defined as a characteristic point P(2) when a minimum point on the apexcardiogram waveform within the range of 30 ms (milliseconds) before and after the peak point of the QRS complex of the fundamental waveform exists (the minimum point is referred to as C1 hereafter); a point corresponding to the peak point of the QRS complex on the apexcardiogram waveform is defined as a characteristic point P(2) when C1 is unclear; a positive vertex on the apexcardiogram waveform within a range of 160 ms before the peak point of the QRS complex in time phase (the same holds true hereafter) is defined as a characteristic point P(1); a positive vertex on the apexcardiogram from 50 ms to 150 ms after the P(2) is defined as a characteristic point P(3); a positive vertex on the apexcardiogram waveform nearest to the IIA sound of the phonocardiogram, which is a sound of the aortic valve closure on the phonocardiogram, within a range of less than 60 ms before the IIA sound of the phonocardiogram is defined as a characteristic point P(5); regarding P(5), the case where the P(5) is represented by specifying the closest positive vertex to the IIA sound of the phonocardiogram within a range of less than 40 ms before the IIA sound of the phonocardiogram is then defined as a characteristic point P' (5); the case where the P(5) is represented by specifying the closest positive vertex to the IIA sound of the phonocardiogram from 40 ms to less than 50 ms before the IIA sound of the phonocardiogram is identified as a characteristic point P"(5); and the case where the P(5) is represented by specifying the closest positive vertex to the IIA sound of the phonocardiogram from 50 ms to less than 60 ms before the IIA sound of the phonocardiogram is identified as a characteristic point P"' (5) (any of the characteristic points P(5), P'(5), P"(5) and P"'(5) are also specified or collectively referred to as P(5) hereafter, except for the case where P'(5), P"(5) and P"'(5) are particularly differentiated); a positive vertex on the apexcardiogram waveform from the point 150 ms after the P(2) to the point 60 ms before the IIA sound of the phonocardiogram is defined as a characteristic point P(4); a negative extreme value on the apexcardiogram waveform from 50 ms to 150 ms after the IIA sound of the phonocardiogram is defined as a characteristic point P(6); and a positive vertex on the apexcardiogram waveform from 100 ms to 240 ms after the IIA sound of the phonocardiogram and after the P(6) when the P(6) exists is defined as a characteristic point P(7). The characteristic points P(1), P(2), P(3), P(4), P(5) (P' (5), P" (5), P"' (5)), P(6), and P(7) are defined as a first characteristic-point group; a waveform risen before the P(1), which is a positive vertex on the apexcardiogram, is defined as a wave A; a waveform risen from the P(2) and having a positive extreme on the apexcardiogram is defined as a wave E; and an ascending wave starting from the P(6) is defined as a wave F; a positive peak position of the wave A of the first-order differential waveform of the apexcardiogram is defined as a point a; a positive peak position of the wave E is defined as a point e; a positive peak position of the wave F is defined as a point f; and heights of the a, the e, and the f points are defined as a, e, and f, respectively; a first characteristic-point evaluating means is defined as an evaluating means for evaluating existence of at least two characteristic points in the first characteristic-point group; a second characteristic-point evaluating means is defined as an evaluating means for evaluating height of at least one of the P(1), the P(2), and the P(7) when ordinate value of the lowest position of the fundamental waveform is defined as zero and when the maximum coordinate value of the fundamental waveform is normalized to 1000 points; a third characteristic-point evaluating means is defined as an evaluating means for defining, regarding time of each of the characteristic points, P(2)-P(3) time (time from the P(2) to the P(3), the same holds true hereafter), ratio of P(3)-P(5) time to P(2)-P(6) time, P(6)-P(7) time, 2-P(6) time (time from the IIA sound of the phonocardiogram to the P(6), the same holds true hereafter), and 2-P(7) time as characteristic factors, and evaluating the value of at least one of the characteristic factors; a first waveform evaluating means is defined as an evaluating means for comparing to a waveform evaluation pattern stored in the biomedical information measuring device or waveform evaluation patterns of the apexcardiogram input to the biomedical information measuring device from outside thereof so as to evaluate type of the fundamental waveform; a second waveform evaluating means is defined as an evaluating means for evaluating values of the a, the e, and the f; a third waveform evaluating means is defined as an evaluating means for evaluating existence of a section where it can be determined that there is a differential waveform running almost horizontally near a point having a derivative value of zero between the point e of the first-order differential waveform of the apexcardiogram and the lowest point immediately before the point f; a fourth waveform evaluating means is defined as an evaluating means for evaluating whether the lowest point immediately before the point f of the first-order differential waveform of the apexcardiogram falls within the first half of the section between the point having a derivative value of zero immediately before the lowest point and the point having a derivative value of zero immediately after the lowest point; a fifth waveform evaluating means is defined as an evaluating means for evaluating whether the apexcardiogram is a monomodal graph not having P(5) but P(3) (referred to as monomodal graph hereafter), and in the case where there is the monomodal graph, evaluating whether the width of the graph in the section after P(3) in time phase (referred to as hemi-posterior width of the P3) at 700 points when the height of P(3) is normalized to 1000 points is less than 100 ms; and a sixth waveform evaluating means is defined as an evaluating means for extracting characteristics of the first-order differential waveform from the time of the IIA sound of the phonocardiogram in the first-order differential waveform of the apexcardiogram to the vicinity of the P(6) and evaluating medical information from those characteristics. It is also preferable that a data processing means has at least one of the nine evaluating means of the first to the third characteristic-point evaluating means and the first to the sixth waveform evaluating means, and a health condition evaluating means for the living body to be measured.

Particularly, with the first characteristic point evaluating means, the present invention can obtain medical information of the living body to be measured from the existence of at least two of the characteristic points P(1)-P(2), P(2), P(3), P(4), P(5) (P'(5), P"(5), P"'(5)), P(6) and P(7), as described before.

The inventor of the present invention has reached the invention of the biomedical information measuring method for the biomedical information measuring device and the biomedical information measuring system having a characteristic point analyzing algorithm given below, for example, as a result of examining both medical knowledge of the inventor and results of measuring an apexcardiogram of a living body to be measured based on the present invention, analyzing data based on the present invention, and making diagnosis in combination with various conventional measuring means aside from the present invention for verification thereof.

An exemplary preferred evaluating method is given below.

First, evaluation is made according to existence or nonexistence of the characteristic points P(3) to P(5).

When the P(3) and the P'(5) exist, possibility of left ventricular systolic dysfunction is very low. When the P(3) and the P"(5) exist, possibility of left ventricular systolic dysfunction is low. When the P(3) and the P"'(5) exist, there is a chance of left ventricular systolic dysfunction.

In the case of a monomodal wave where the P(4) exists but the P(3) and the P(5) do not, either possibility of left ventricular systolic dysfunction is high or there is possibility of left ventricular hypertrophy without being associated with systolic dysfunction.

In the case of a monomodal waveform where the P(3) exists but the P(5) does not, there is a possibility of either left ventricular systolic dysfunction or left ventricular hypertrophy when width of the graph at 700 points when height of the P(3) is normalized at 1000 points, namely a portion behind the P(3) in time phase (referred to as hemi-posterior width of the P3) is 100 ms or greater.

Note that when mixed data of bimodal data where the P(3) and the P(5) exist and monomodal waveform data where the P(3) exists but the P(5) does not of measured data of the same period of the same living body to be measured, it is preferable to make evaluation using the bimodal data as a rule.

Taking a look at height (longitudinal axis) of the P(1), P(2) and P(7) next is preferred. In the case where the P(7) exists, possibility of left ventricular early diastolic dysfunction is high when the P(7) is equal to or higher than the P(2). On the apexcardiogram where the lowest point on an intensity axis (longitudinal axis) is set to zero points and a highest point is normalized to 1000 points, when the P(7) is less than the P(2) and the P(7) is equal to or greater than 50 and less than 200, possibility of normal left ventricular diastolic function in an early stage is high. In the case where the P(7) is less than 50, possibility of a state where expansion of the left ventricle in the early diastolic phase does not occur easily is high. In the case where the P(7) is equal to or greater than 200, there is a possibility of left ventricular diastolic dysfunction with an absolutely increased load due to increased transmitral blood flow in the early diastolic phase. Moreover, there is also a possibility of relatively increased load in the early diastolic phase where there is an increased load without increased transmitral blood flow when the left ventricular muscle itself is injured. However, when a young person has normal left ventricular systolic function and good compliance of the left ventricle, the P(7) may be 200 or greater.

In the case where the P(7) does not exist, the apexcardiogram waveform slowly rises from the P(6), and it is evaluated that the wave F is not recognized in the apexcardiogram waveform not showing a critical point. It is evaluated in this case that neither the P(7) nor the point f exist.

In the case where the P(7) does not exist, possibility of diastolic dysfunction where dilation of the left ventricle in the early diastolic phase does not occur easily is high.

Time phases of the characteristic points P(1) to P(7) are explained next.

When the P(2)-P(3) time is equal to or greater than 50 ms and less than 125 ms, it is evaluated as normal left ventricular systolic function, and when it is 125 to less than 150 ms, there is a possibility of either left ventricular systolic dysfunction or left ventricular hypertrophy, thereby evaluated as caution needed.

Regarding the P(3)-P(5) time and the P(2)-P(6) time, If the P(3)-P(5) time is 45% or greater than the P(2)-P(6) time, it should be evaluated to have a high possibility that left ventricular function (systolic function and diastolic function) is normal, otherwise if it is 40% or greater and less than 45%, it should be evaluated to have a high possibility that left ventricular function is normal, and yet otherwise if it is under 40%, it should be evaluated to have a high possibility that left ventricular function (at least either systolic function or diastolic function) is abnormal.

If the P(6)-P(7) time is less than 100 ms, it should be evaluated to have a high possibility of normal left ventricular early diastolic function, and otherwise if it is equal to or greater than 100 ms and less than 150 ms, it should be evaluated to have a high possibility of left ventricular early diastolic dysfunction.

If the 2-P(6) time is less than 150 ms, it should be evaluated to have a high possibility of normal left ventricular early diastolic function, and otherwise if it is equal to or greater than 150 ms and less than 200 ms, it should be evaluated to have a high possibility of left ventricular early diastolic dysfunction. Note that the first negative extreme value at 200 ms or more away from the IIA sound of the phonocardiogram is not determined as the P(6).

If the 2-P(7) time is less than 240 ms, it should be evaluated to have a high possibility of normal left ventricular early diastolic function, and otherwise if it is 240 ms or greater, it should be evaluated to have a high possibility of left ventricular early diastolic dysfunction.

Magnitude of a first-order differential waveform in an apexcardiogram is explained next.

Left ventricular end diastolic function is evaluated to be normal when 'a' of a first-order differential waveform is less than e/4, otherwise caution is needed of the left ventricular end diastolic function when 'a' is equal to or greater than e/4 and less than e/2, and it is evaluated to have left ventricular end diastolic dysfunction when 'a' is equal to or greater than e/2.

For 'f', it is evaluated to have normal left ventricular early diastolic function when 'f' is less than e/2, otherwise caution is needed of the left ventricular early diastolic function when 'f' is equal to or greater than e/2 and less than 2e/3, and it is evaluated to have left ventricular early diastolic dysfunction when 'f' is equal to or greater than 2e/3. When 'a' is lower than the point f, the case of normal left ventricular early diastolic function and particularly very good left ventricular diastolic function and the opposite case of severe heart failure (decompensated heart failure or severely decreased left ventricular diastolic function) may be considered.

Carrying out at least a part of the evaluations of abnormalities allows evaluation of cardiac functions of portions that could not be evaluated thus far, whereby evaluation or determination of normal, caution needed and abnormal, for example, can be displayed and even informed to the living body to be measured.

Moreover, a second-order differential of the apexcardiogram waveform may be used in extraction of characteristic points of the first-order differential.

The present invention contributes greatly to display of medical information of the living body to be measured by including added functions not available until now to the ultrasonograph as a substitute for palpation, by which vigor and strength of complicated heart motions will be felt.

More reliable medical information may be obtained by using ECG analysis software and phonocardiogram software in analysis of the apexcardiogram than when merely analyzing only data of the apexcardiogram. With the present invention, apart from normal analysis, a specially established analyzing means is used to analyze an apexcardiogram of the case where there are conduction disturbances such as complete left bundle branch block or the like, and arrhythmias such as atrial fibrillation or the like. Classification of accurate medical information is possible by taking data of a living body to be measured for which state of disease is already known, creating a characteristics map (e.g., correspondence chart of data and state of disease) or a waveform evaluation pattern, for example, and transferring it to a predetermined analysis program so as to analyze it in order to respond to such examples.

In the case of an apexcardiogram, normalization of data such that the maximum value of pulsation amplitude, for example, becomes 1000 taking into consideration change in detection output due to position or way of placing an ultrasound probe on a living body makes it easy to evaluate. Note that an example of normalizing a single waveform and example of focusing on a single waveform of a plurality of normalized waveforms are examples of measuring an apexcardiogram according to the present invention.

Furthermore, when measuring an apexcardiogram using the ultrasound probe of the present invention, measured data for some characteristic points such as the characteristic points P(1), P(2), P(6) and P(7) may become unclear due to conditions of the living body to be measured. This may be caused by several things such as way of placing the sensor against the body, data processing method, noise state of the measuring device, condition of the living body, etc. In such a case, with the present invention, it is possible to create a data processing software based on many measurement results, including noise elimination, apexcardiogram selection, superposition and correction, and obtain an apexcardiogram usable for medical examination.

Amplified data is stored in memory and input to a waveform rendering/graphics rendering device, a graph is created when the data, for example, is for an apexcardiogram, an ECG or a phonocardiogram, graphics rendering is carried out when the data is for amplitude distribution or strength distribution of pulsations, and characteristic extraction is carried out using results thereof. Characteristic extraction includes, for example, extraction of characteristics such as extraction of extreme values in the graph of an apexcardiogram, comparison of magnitude of extreme values, and extraction of extreme values of the first-order differential waveform and the second-order differential waveform, waveforms of the apexcardiogram, etc. At least relevant parts of the measured data are stored in memory as digital data.

Data from which characteristics have been extracted is stored in the memory as detected data and is evaluated for the extracted waveform, characteristics, etc. When the graph and figure resulting from the waveform rendering and graphics rendering are evaluated as insufficient as a result of evaluating whether the graph and figure are sufficient to diagnosis of the living body to be measured or when wanting to change processing conditions for research, etc., new or changed amplitude and noise elimination conditions or characteristic extraction conditions are set and data processing is carried out.

When the extracted waveform, characteristics, etc. are evaluated as sufficient, a first determination or evaluation of the medical information is carried out so as to create detected data such as biomedical information, etc. In addition to using information other than information based on this measured data such as blood pressure or drug dosage information, a second determination or evaluation of the medical information is carried out as needed.

When it is evaluated from the aforementioned respective processing that further examination is necessary, processing proceeds to a predetermined data processing step so as to carry out further determination or evaluation.

Moreover, the data processing, determination and evaluation may be carried out automatically or semi-automatically in accordance with a program or the like, diagnosis and evaluation by a medical specialist is possible, and a combination thereof is also possible.

When it is evaluated by the first and the second determination or evaluation that there is a problem in creation of detected data such as biomedical information, in a processing condition setting step, conditions for waveform rendering and graphics rendering are set, and waveform rendering and graphics rendering are carried out so as to create medical information.

Storing time course information such as detected data of the same living body to be measured, for example, statistical data that serves as a reference in diagnosis etc., a model pattern of an apexcardiogram or the like in a reference data memory unit in the memory allows implementation of further objective and accurate evaluation.

Second evaluation information is stored in the memory and displayed on the display device as needed so as to create medical information of the living body to be measured. The medical information of the living body to be measured is displayed on the display device as needed.

Display on the display device is not limited hereto, and an apexcardiogram, medical information, etc. may be displayed on the display device as needed in each case. Moreover, a display screen may be configured so as to classify a plurality of portions and display them simultaneously so as to be able to look over results of a plurality of analyzing steps. Such a configuration allows further accurate and speedy evaluation by the present device or user of the present device.

While an apexcardiogram itself is naturally important information in characteristic extraction and evaluation of the apexcardiogram, differential data of the apexcardiogram also plays a very important role, as is evident from the embodiments of the present invention. For example, in the case of extracting characteristics or evaluating characteristics in accordance to a program, making an automatic evaluation or semi-automatic evaluation, or a medical doctor interpreting an apexcardiogram that is difficult to estimate, utilization of first-order differential and second-order differential data allows easy characteristic extraction, evaluation thereof, and diagnosis in most situations. It is preferable to have a function for recognizing apexcardiogram waveforms.

The measuring method and the data processing method described in the embodiments of the present invention allow many variations. For example, an apexcardiogram as a graph obtained as a result of graphics rendering changes if noise elimination conditions are varied. What kind of time and what kind of processing conditions are appropriate greatly influences accuracy of evaluation of the device. The biomedical information measuring device according to the embodiment of the present invention limiting these conditions in more detail is included as part of the invention. As apexcardiograms, according to purpose, there is a case of adopting successive apexcardiograms nearest to the fibrous pericardium, for example, between the fibrous pericardium and the body surface, case of adopting an apexcardiogram having the highest reflection intensity, and case of accumulating reflected waves between the fibrous pericardium and the body surface.

The ultrasound transmitters and the receiving sensors of the ultrasound probe of the present invention are mounted as probes with a plurality of ultrasound transmitting elements and receiving elements arranged in parallel, and used by controlling actions of the transmitting elements and receiving elements through electronic scanning. A thin probe having the respective elements aligned one-dimensionally may be used, as described before; however, by using those that have the respective elements aligned two-dimensionally and mounted as probes, it is possible to carry out accurate measurement, and additionally to measure motion per pulsation of the cardiac apex, which enables information obtained through palpation to be obtained more accurately, and to clarify motion of the left ventricle that has been unclear until now.

With the embodiments of the present invention, the respective transmitting elements and receiving elements, which are the same elements, may be used for transmission and reception through a scanning operation. Moreover, depending on purpose of measurement, they may be prepared independently from each other and mounted together. Alternatively, depending on purpose of measurement, they may be prepared independently from each other and mounted separately.

Further alternatively, as described before, a sensor (probe) used as a probe on the conventional ultrasonograph may be used as the ultrasound probe of the present invention. For measurement of the apexcardiogram according to the present invention, in a preferred embodiment, an ultrasound transmitting element and an ultrasound receiving element are provided on the ultrasound probe, an ultrasound wave transmitted from the oscillating element hits the cardiac apex and/or the vicinity thereof, the reflected wave is received by the receiving element, and an apexcardiogram is then measured. To give an example, an apexcardiogram is measured using 192 ultrasound transducers, wherein each of twelve-transducer groups transmits to scan in turn one after another, and a time is prepared for the same element to function as an oscillating element and to function as a receiving element by controlling timings so as to receive the reflected ultrasound wave from the cardiac apex and/or the vicinity thereof.

Moreover, as another example of the present invention, an ultrasound transmitting element and an ultrasound receiving element are provided as ultrasound probes, the ultrasound transmitting element transmits an ultrasound wave of a single kind of wavelength from a single oscillating element, the transmitted ultrasound wave from the same oscillating element that has hit the cardiac apex and/or the vicinity thereof and reflected therefrom is received by the ultrasound receiving element having a receiver or made of multiple receiving elements arranged adjacently and capable of independently receiving waves, and an apexcardiogram is then measured. In the case of using multiple kinds of ultrasound waves as pulsation-measuring signals, it is preferable to use an ultrasound oscillating element for each type.

When measuring an apexcardiogram using ultrasound waves, many kinds of technology used in the electronic engineering field, such as technology of modulating frequency, amplitude, or phase of the ultrasound waves and amplifying the modulated signal as a reference signal may be used.

Not only has there not been an idea of using ultrasound waves in measurement of an apexcardiogram conventionally, use of functions of the conventional ultrasonograph also allows more accurate evaluation of state of the cardiac function, and use thereof in carrying out verification of evaluation results of health condition according to measurement of the apexcardiogram, thereby allowing further extensive examination.

As can be understood from the above respective embodiments, the apexcardiogram as an example of biomedical information recorded according to the present invention provides much information on the health condition of the living body to be measured.

In the example of an apexcardiogram of a healthy person in which the characteristic points P(3), P(5) and P(6) exist, the first-order differential waveform from the characteristic point P(2) to either the P(6) or the P(7) precipitously passes from a derivative value maximum point to a point where derivative value is nearly zero (referred to as zero point 1 hereafter) generally in a straight line and then passes through a negative portion, changes to nearly horizontal and then passes through a positive portion, reaches a point where derivative value is nearly zero (referred to as zero point 2 hereafter), drops down from the zero point 2 to a derivative value minimum point (referred to as minimum point A hereafter) generally in a straight line, rises generally in a straight line, and passes through a point where derivative value is nearly zero (zero point 3), reaching the point f. A straight line 1, which connects the derivative value maximum point and the zero point 1 by a straight line, a straight line 2, which connects the zero point 1 and the zero point 2 by a straight line, and a straight line 3, which connects the zero point 2 and the minimum point A by a straight line are assumed.

On the other hand, in the example of an apexcardiogram of a living body to be measured having abnormal symptoms, which is different from the healthy person, where the characteristic points P(3) and P(5) do not exist but the P(4) does, the first-order differential waveform from the characteristic point P(2) to either the P(6) or the P(7) passes from a derivative value maximum point to a point where derivative value is nearly zero (zero point 4) generally in a straight line, and then reaches a point where derivative value is nearly zero (referred to as zero point 2 hereafter), drops down to a derivative value minimum point (referred to as minimum point B hereafter) generally in a straight line without changing to nearly horizontal as in the case of the healthy person, and then rises generally in a straight line, reaching a point that is nearly zero (zero point 5).

Let us focus on the lateral axis (time axis) of the first-order differential waveform in the example of the apexcardiogram of a healthy person.

The zone from the characteristic point P(5) to P(6), namely zone nearly corresponding to the isovolumic diastolic phase corresponds to a zone including the zero point 2 and the zero point 3 of the first-order differential waveform. It is considered that the better the extensibility of the left ventricular muscles in the diastolic phase, the sooner they relax. In other words, a relaxation peak is considered to appear in the early stage. In the apexcardiogram, relaxation begins from the P(5). The fact that the minimum point A indicating a maximum rate of change is positioned in the front portion of the zone between the zero point 2 and the zero point 3 in the first-order differential waveform that shows rate of change of the relaxation can be said to indicate that relaxation in the aforementioned normal example will occur in the early stage. In this manner, left ventricular diastolic hemodynamics in the isovolumic diastolic phase can be distinctively and easily evaluated.

At the same time, in the apexcardiogram of the person with abnormality, the minimum point B is positioned in the back portion of a zone between the zero point 4 and the zero point 5.

The P(1)-P(2) and the point a are indicators of left ventricular end diastolic dysfunction. Since the P(1)-P(2) and the point a are relevant, pathophysiology can be classified in the following manner when relationships to one another of these two items are studied. In the case of type 1 and type 2, left atrial hypercontraction and high left ventricular end diastolic pressure are suggested when the P(1)-P(2) is high and the point a is high. High left ventricular end diastolic pressure without any left atrial hypercontraction is suggested when the P(1)-P(2) is high and the point a is low. Left atrial hypercontraction is suggested when the P(1)-P(2) is high and the point a is high. No left ventricular end diastolic load is suggested when the P(1)-P(2) is low and the point a is low.

As with type 3, the case where the P(1) and the P(2) are identical suggests that left ventricular end diastolic dysfunction is mild when the point a is low. Left atrial hypercontraction and high left ventricular end diastolic pressure are suggested when the point a is high.

With the biomedical information measuring device according to the present invention, once basic data of the apexcardiogram is measured and loaded into the device, medical information of the living body to be measured may be evaluated, recorded and displayed as necessary using items regarding the respective characteristic points described above.

While knowing the amplitude or distribution of amplitude of the apexcardiogram or the apex beat may be given for grasping motion of the heart, in addition, for example, knowing intensity or intensity distribution of driving such as whether the heart is moving vigorously or whether it is moving strongly may also be given. As is well-known by medical doctors who perform palpation, the fact that the heart makes complex motions and intensity and intensity distribution of pulsations felt through palpation as vigor and strength of the motions of the heart can be detected using the ultrasound probe according to the present invention is important information in making accurate evaluation.

The embodiments may be applied in various ways as needed and can bring about results that could not be expected in the past. For example, once an apexcardiogram is measured, results may be explained to the patient promptly and the apexcardiogram may also be shown and explained to the patient. Moreover, previous data of the living body to be measured, statistical data useful in diagnosis of the living body to be measured, etc. as a kind of reference data is input to the device of the present invention, and therefore rating of the health condition of the living body to be measured, understanding of change in the health condition, and explanation of it to the living body to be measured so as to perform effective health management may be carried out.

Various sensors such as a wave sensor may be incorporated in the ultrasound probe. Such a configuration not only allows the possibility of making the measuring device highly accurate, simple, small-sized, low cost, etc., but also great alleviation of mental load due to mounting of many sensors on the living body to be measured so as to measure.

By giving the ultrasound probe a three-dimensional configuration, pressure, heart sounds, etc. can be detected and an S/N ratio of measured data can be increased.

The device of the present invention may be configured by incorporating a transmitting means or a transmitting and receiving means to a pulsation sensor of the various configurations described above and mounting it on a living body to be measured, or mounting a pulsation sensor and a small transmitter or a transceiver on the living body to be measured so as to transmit or transmit and receive measured data etc. from the sensor to an external device carried by the living body to be measured or an external device at a distance from the living body to be measured. The device may also be useful in health management by the user him/herself, and allow diagnosis and health management of the living body to be measured by a medical specialist.

In measuring an apexcardiogram, even when sensors for an ECG and a phonocardiogram are provided on the device of the present invention, and even when data for the ECG and the phonocardiogram is measured individually from the device of the present invention and input to the device of the present invention, it is important to synchronize the apexcardiogram to at least either the ECG or the phonocardiogram. This allows evaluation with greater reliability.

As a result of detailed examination, it is found that an analyzing method of characteristic points etc. in compliance with the algorithm of the present invention can be applied to the apexcardiogram measured using the biomedical information measuring device as an embodiment of the present invention. Moreover, since the biomedical information measuring device of the present invention has a function as a conventional ultrasonograph in addition to an apexcardiogram measuring function, medical information of the living body to be measured may be extracted by adding diagnosis from measured data as an ultrasonograph to diagnosis from an apexcardiogram, and medical information of the living body to be measured may be extracted with even greater reliability.

As described above, the present invention brings about extremely great results such as implementing a small and low-cost measuring device, making it possible for a medical doctor to measure an apexcardiogram at a hospital outpatient clinic or in a hospital room, bringing about extremely great results for medical examination of being able to give feedback to the patient on the spot, increasing quality of medical examination skills of inspection, palpation and auscultation, and making it possible to accurately diagnose cardiovascular diseases. Moreover, it demonstrates extremely great results even in clinical medical education for medical students, medical residents and life-education for medical doctors. Furthermore, the present invention may be widely applied to medical specialized knowledge such as normal health management, remote health management, and the like.

Examples of electronic components of the present invention have been described with reference to the embodiments; however, the present invention is not narrowly limited to only the above embodiments. For example, while the present invention has been described by giving data of the case of a person in order to show that the living body to be measured exhibits extremely great results in the case of a person, it is not limited to the examples according to the technical ideas, and many variations are possible based on the technical ideas of the present invention.

### Industrial Applicability

The present invention remarkably improves the level of examining cardiovascular diseases, increases collaborative results with other medical fields, makes great contributions to development in medical science, and brings about large economical results, such as greatly contributing to economizing health care costs. The present invention is widely available in the medical field and medical education field, and is also widely available in the health-care equipment field.

## Claims

1. A biomedical information measuring device, comprising an ultrasound probe for an apexcardiogram capable of measuring an apexcardiogram, which comprises an ultrasound transmitter that makes an ultrasound signal enter from a body surface of a chest wall of a living body to be measured to the cardiac apex and/or vicinity of the cardiac apex, and a receiving sensor for measuring a reflected ultrasound signal from each part in the living body to be measured of the ultrasound signal; said biomedical information measuring device further comprising a means for creating an apexcardiogram; an evaluating means for evaluating characteristics of the measured apexcardiogram; and a displaying means for displaying the apexcardiogram.

2. The biomedical information measuring device according to claim 1, wherein in the evaluating means, an apexcardiogram waveform of one beat of the living body to be measured is defined as a fundamental waveform, that is, as an example, when the apexcardiogram waveform ranging from a predetermined time before a point (referred to as peak point of the QRS complex hereafter), which corresponds to each positive peak point (R) of respective QRS complex of an ECG at the same time as the apexcardiogram of the same living body to be measured, for example, a lead II ECG in a standard 12 leads electrocardiogram to the predetermined time before a subsequent peak point of the QRS complex is defined as the fundamental waveform, and heart rate compensating coefficient is set to √V/60 (√V/60 means square root of V/60) at a time on a time axis of the apexcardiogram waveform where V denotes heart rate per minute when the fundamental waveform is represented by time on the lateral axis and amplitude of the apexcardiogram waveform on the longitudinal axis; and the time is multiplied by √V/60 and the resulting value is defined as a converted value for the heart rate of 60 on the time axis of the following apexcardiogram; C1 is defined as a characteristic point P(2) when a minimum point on the apexcardiogram waveform within the range of 30 ms (milliseconds) before and after the peak point of the QRS complex of the fundamental waveform exists (the minimum point is referred to as C1 hereafter); a point corresponding to the peak point of the QRS complex on the apexcardiogram waveform is defined as a characteristic point P(2) when C1 is unclear; a positive vertex on the apexcardiogram waveform within a range of 160 ms before the peak point of the QRS complex in time phase (the same holds true hereafter) is defined as a characteristic point P(1); a positive vertex on the apexcardiogram from 50 ms to 150 ms after the P(2) is defined as a characteristic point P(3); a positive vertex on the apexcardiogram waveform nearest to the IIA sound of the phonocardiogram, which is a sound of the aortic valve closure on the phonocardiogram, within a range of less than 60 ms before the IIA sound of the phonocardiogram is defined as a characteristic point P(5); regarding P(5), the case where the P(5) is represented by specifying the closest positive vertex to the IIA sound of the phonocardiogram within a range of less than 40 ms before the IIA sound of the phonocardiogram is then defined as a characteristic point P' (5); the case where the P(5) is represented by specifying the closest positive vertex to the IIA sound of the phonocardiogram from 40 ms to less than 50 ms before the IIA sound of the phonocardiogram is identified as a characteristic point P"(5); and the case where the P(5) is represented by specifying the closest positive vertex to the IIA sound of the phonocardiogram from 50 ms to less than 60 ms before the IIA sound of the phonocardiogram is identified as a characteristic point P"' (5) (any of the characteristic points P(5), P'(5), P"(5) and P"'(5) are also specified or collectively referred to as P(5) hereafter, except for the case where P'(5), P"(5) and P"'(5) are particularly differentiated); a positive vertex on the apexcardiogram waveform from the point 150 ms after the P(2) to the point 60 ms before the IIA sound of the phonocardiogram is defined as a characteristic point P(4); a negative extreme value on the apexcardiogram waveform from 50 ms to 150 ms after the IIA sound of the phonocardiogram is defined as a characteristic point P(6); and a positive vertex on the apexcardiogram waveform from 100 ms to 240 ms after the IIA sound of the phonocardiogram and after the P(6) when the P(6) exists is defined as a characteristic point P(7); wherein the characteristic points P(1), P(2), P(3), P(4), P(5), P' (5), P" (5), P"' (5), P(6), and P(7) are defined as a first characteristic-point group; a waveform risen before the P(1), which is a positive vertex on the apexcardiogram, is defined as a wave A; a waveform risen from the P(2) and having a positive extreme on the apexcardiogram is defined as a wave E; and an ascending wave starting from the P(6) is defined as a wave F; a positive peak position of the wave A of the first-order differential waveform of the apexcardiogram is defined as a point a; a positive peak position of the wave E is defined as a point e; a positive peak position of the wave F is defined as a point f; and heights of the a, the e, and the f points are defined as a, e, and f, respectively; a first characteristic-point evaluating means is defined as an evaluating means for evaluating existence of at least two characteristic points in the first characteristic-point group; a second characteristic-point evaluating means is defined as an evaluating means for evaluating height of at least one of the P(1), the P(2), and the P(7) when ordinate value of the lowest position of the fundamental waveform is defined as zero and when the maximum coordinate value of the fundamental waveform is normalized to 1000 points; a third characteristic-point evaluating means is defined as an evaluating means for defining, regarding time of each of the characteristic points, P(2)-P(3) time (time from the P(2) to the P(3), the same holds true hereafter), ratio of P(3)-P(5) time to P(2)-P(6) time, P(6)-P(7) time, 2-P(6) time (time from the IIA sound of the phonocardiogram to the P(6), the same holds true hereafter), and 2-P(7) time as characteristic factors, and evaluating the value of at least one of the characteristic factors; a first waveform evaluating means is defined as an evaluating means for comparing to a waveform evaluation pattern stored in the biomedical information measuring device or waveform evaluation patterns of the apexcardiogram input to the biomedical information measuring device from outside thereof so as to evaluate type of the fundamental waveform; a second waveform evaluating means is defined as an evaluating means for evaluating values of the a, the e, and the f; a third waveform evaluating means is defined as an evaluating means for evaluating existence of a section where it can be determined that there is a differential waveform running almost horizontally near a point having a derivative value of zero between the point e of the first-order differential waveform of the apexcardiogram and the lowest point immediately before the point f; a fourth waveform evaluating means is defined as an evaluating means for evaluating whether the lowest point immediately before the point f of the first-order differential waveform of the apexcardiogram falls within the first half of the section between the point having a derivative value of zero immediately before the lowest point and the point having a derivative value of zero immediately after the lowest point; a fifth waveform evaluating means is defined as an evaluating means for evaluating whether the apexcardiogram is a monomodal graph not having P(5) but P(3) (referred to as monomodal graph hereafter), and in the case where there is the monomodal graph, evaluating whether the width of the graph in the section after P(3) in time phase (referred to as hemi-posterior width of the P3) at 700 points when the height of P(3) is normalized to 1000 points is less than 100 ms; and a sixth waveform evaluating means is defined as an evaluating means for extracting characteristics of the first-order differential waveform from the time of the IIA sound of the phonocardiogram in the first-order differential waveform of the apexcardiogram to the vicinity of the P(6) and evaluating medical information from those characteristics; wherein a data processing means has at least one of the nine evaluating means of the first to the third characteristic-point evaluating means and the first to the sixth waveform evaluating means, and a health condition evaluating means for the living body to be measured.

3. The biomedical information measuring device according to claim 2, wherein: of evaluation results from the evaluating means, an evaluation result 1 denotes that possibility of left ventricular systolic dysfunction is very low when the P(3) and the P'(5) exist; an evaluation result 2 denotes that possibility of left ventricular systolic dysfunction is low when the P(3) and the P"(5) exist; an evaluation result 3 denotes that there is a chance of left ventricular systolic dysfunction when the P(3) and the P"'(5) exist; and an evaluation result 4 denotes that there is a possibility of either left ventricular systolic dysfunction or left ventricular hypertrophy in the case of a monomodal waveform having the P(4) but not having the P(3) and the P(5) and when width of the graph at 700 points when height of the P(3) is normalized at 1000 points, namely a portion behind the P(3) in time phase (referred to as hemi-posterior width of the P3) is 100 ms or greater; and any one of the evaluation results 1 to 4 is displayed.

4. The biomedical information measuring device according to any one of claim 1 to claim 3, wherein the means for creating the apexcardiogram and the means for displaying are a means for making an apexcardiogram converted to a single line drawing by a data processing unit built into the device and/or externally attached to the device, and a means for displaying, respectively.

5. The biomedical information measuring device according to any one of claim 1 to claim 4, wherein the apexcardiogram is made up of the continuously curved lines where ultrasound reception intensity is strongest between the fibrous pericardium and the body surface.

6. The biomedical information measuring device according to any one of claim 1 to claim 4, wherein the apexcardiogram is made up of a curved line of accumulated reflection intensity of reflected ultrasound waves in a cross section between the fibrous pericardium and the body surface.

7. The biomedical information measuring device according to any one of claim 1 to claim 6, wherein the ultrasound probe for measuring the apexcardiogram can transmit at least two kinds of ultrasound signals of different frequencies.

8. The biomedical information measuring device according to any one of claim 1 to claim 7, wherein central rays of ultrasound beams that are transmitted from multiple transducers or multiple oscillating elements (referred to as multiple first transducers) mainly constituting an ultrasound transmitter of the ultrasound probe for measuring the apexcardiogram are in parallel with each other, and the multiple first transducers are arranged adjacently next to each other.

9. The biomedical information measuring device according to any one of claim 1 to claim 8, wherein the ultrasound probe includes a plurality of different ultrasound probes.

10. The biomedical information measuring device according to claim 9, wherein the multiple ultrasound probes can be changed over.

11. The biomedical information measuring device according to claim 9 or claim 10, wherein the multiple ultrasound probes have different frequencies with each other.

12. The biomedical information measuring device according to any one of claim 1 to claim 11, further comprising a means for detecting a rib.

13. The biomedical information measuring device according to any one of claim 1 to claim 12, further comprising a means for detecting contact pressure on the probe.

14. The biomedical information measuring device according to any one of claim 1 to claim 13, further comprising a means for changing method of electronic scanning of the respective transducers or oscillating elements constituting the ultrasound probe.

15. The biomedical information measuring device according to any one of claim 1 to claim 14, wherein the ultrasound probe comprises a linear probe.

16. The biomedical information measuring device according to any one of claim 1 to claim 15, further comprising a means for changing refractive index of an acoustic lens member of the probe.

17. The biomedical information measuring device according to any one of claim 1 to claim 16, wherein ultrasound frequency is 5 MHz or greater.

18. The biomedical information measuring device according to any one of claim 1 to claim 17, said biomedical information measuring device has a function of recognizing evaluation results of an apexcardiogram.

19. The biomedical information measuring device according to any one of claim 1 to claim 18, further comprising a means for detecting position of the fibrous pericardium of a living body.

20. The biomedical information measuring device according to any one of claim 1 to claim 19, further comprising a waveform classifying means for classifying types of the fundamental waveform included in the measured data expressed as an apexcardiogram, and a means for displaying or outputting number of respective classified types of waveforms.

21. The biomedical information measuring device according to any one of claim 1 to claim 20, wherein detailed analysis of waveforms is conducted for selected fundamental waveforms based on fundamental waveform classification results of the waveform classification means or input information from outside of the data processing means.

22. An ultrasound probe that can be used in a biomedical information measuring device, wherein selectable multiple probes are incorporated into the ultrasound probe.

23. An ultrasound probe that can be used in a biomedical information measuring device; said ultrasound probe has a configuration allowing use in coupling together to at least two ultrasound probes in a detachable manner so as to measure.

24. An ultrasound probe that can be used in a biomedical information measuring device, wherein the ultrasound probe is flexible.

25. An ultrasound probe that can be used in a biomedical information measuring device, wherein an acoustic lens member capable of changing refractive index having elasticity is arranged between an outer surface of the ultrasound probe on a side that comes into contact with a living body and at least one of multiple first transducers and multiple first receivers.

26. A biomedical information measuring method usable for a biomedical information measuring device or a biomedical information measuring system that are capable of measuring information of the health of a living body; said biomedical information measuring method comprising the steps of: preparing a means for making an ultrasound signal enter from a body surface of a living body to be measured to the cardiac apex and/or vicinity thereof; preparing a means for receiving reflected waves of the ultrasound signal from each part in the living body to be measured; preparing a means for creating an apexcardiogram of the living body to be measured from information of the reflected waves of the ultrasound signal from each part in the living body to be measured; preparing a means for evaluating the apexcardiogram; and preparing a means for displaying the apexcardiogram.
